# EUROPEAN PATENT SPECIFICATION

(11) **EP 2 123 265 B1**
(45) Date of publication and mention of the grant of the patent: **23.05.2012**
(21) Application number: 08009470.9
(22) Date of filing: 23.05.2008
(51) Int. Cl.: A61K 31/09, A61P 17/00, A61P 17/06

(54) **Endipalene formulations in the treatment of psoriasis**
Endipalenformulierungen bei der Behandlung von Psoriasis
Formulations d'endipalène pour le traitement de la psoriasis

(43) Date of publication of application: 25.11.2009
(73) Proprietor: NOVAERA S.r.l., 38100 Trento (IT)
(72) Inventor: Endrici, Giorgio, 38100 Trento (IT)
(74) Representative: Trupiano, Federica

(56) References cited:
- EP-B- 1 011 649
- WO-A-89/10912
- JP-A- 59 122 437
- US-A- 4 229 274
- US-A- 4 885 373
- US-A1- 2002 052 400
- US-A1- 2005 113 483
- US-B1- 6 429 214
- HABERNICKEL V J: "The pharma market as reflected by patents" DRUGS MADE IN GERMANY 2001 DE, vol. 44, no. 1, 2001, pages 25-28, XP008095944 ISSN: 0012-6683
- BLACK ET AL: "Pharmacological and Clinical effects of Lonapalene (RS 43179), a 5-Lipoxygenase Inhibitor, in Psoriasis" JOURNAL OF INVESTIGATIVE DERMATOLOGY, NEW YORK, NY, US, vol. 95, no. 1, 1 January 1990 (1990-01-01), pages 50-54, XP002095380 ISSN: 0022-202X

## Description

### BACKGROUND OF THE INVENTION

Prostaglandins play a major role in the inflammation process, and the inhibition of prostaglandin production, especially production of PGG₂, PGH₂ and PGE₂, has been a common target of antiinflammatory drug discovery. However, common non-steroidal antiinflammatory drugs (NSAIDs) that are active in reducing the prostaglandin-induced pain and swelling associated with the inflammation process are also active in affecting other prostaglandin-regulated processes not associated with the inflammation process. Thus, use of high doses of most common NSAIDs can produce severe side effects, including life threatening ulcers, that limit their therapeutic potential. An alternative to NSAIDs is the use of corticosteroids, which have even more drastic side effects, especially when long term therapy is involved. Previous NSAIDs have been found to prevent the production of prostaglandins by inhibiting enzymes in the human arachidonic acid/prostaglandin pathway including the enzyme cyclooxygenase (COX). The recent discovery of an inducible enzyme associated with inflammation (named "cyclooxygenase-2 (COX-2)" or "prostaglandin G/H synthase II") provides a viable target of inhibition which more effectively reduces inflammation and produces fewer and less drastic side effects.

In another portion of the arachidonic acid pathway, physiologically active leukotrienes, such as leukotriene B₄ (LTB₄), leukotriene C₄ (LTC₄) and leukotriene D₄ (LTD₄) and other metabolites, are produced by the 5-lipoxygenase-mediated (5-LO) oxidation of arachidonic acid.

A wide variety of agents have been reported as 5-LO inhibitors. The majority of the series appear to be lipophilic reducing agents, including phenols, partially saturated aromatics, and compounds containing heteroatom-heteroatom bonds. Many of these are not selective 5-LO inhibitors, but often affect CO and other LOs as well. In vivo systemic activity for many of these has been, in general, disappointing, probably because of poor bioavailability caused by lipophilicity and metabolic instability (oxidation, and conjugation of phenolic compounds).

In the treatment of dermatological disorders related to inflammation, in particular psoriasis, is known the use of lonapalene.

Lonapalene (6-chloro-2,3-dimethoxynaphtalendiacetate) is a known molecule.

The pharmacologic and clinical effects of Lonapalene have been for example determined in a double-blind, placebo-controlled, topical study in ten volunteers with psoriasis. A statistically significant clinical improvement was seen in lesions treated with 2% Lonapalene ointment as compared with vehicle-treated sites.

The above described result as well as the selectivity of the pharmacologic response, suggest that the therapeutic effect of topical Lonapalene in psoriasis may be very interesting. Unfortunately this molecule is not suitable for extensive clinical use, because serious side effects are related to topical application.

In view of the above, other compounds have been studied as alternatives to the use of Lonapalene.

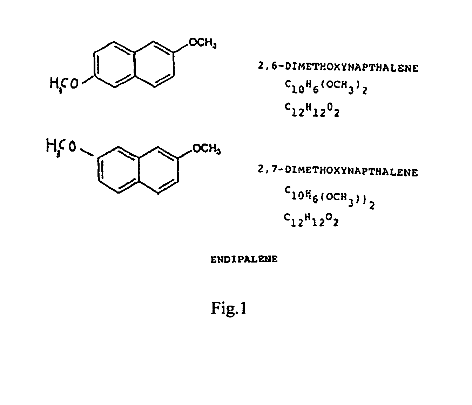

For example, EP 1011649 describes a molecule: 2,6 or 2,7-dimethoxynaphtalene, named Endipalene (Fig.1), that seems to have the same action mechanism of Lonapalene excluding remarkable side effects.

According to EP1011649, Endipalene should be used topically, with a concentration between 1% and 2%.

Nevertheless a continuous need for additional formulations to be used in the treatment of dermatological disorders related to inflammation, in particular psoriasis, exists.

In addition, it would be desirable to have a formulation available, that can be used for a long period of time without showing severe side effects but still maintaining a significant pharmacological activity.

### DESCRIPTION OF THE INVENTION

The present invention relates to different formulations of 2,6-dimethoxynaphtalene or 2,7-dimethoxynaphtalene or their mixture (Endipalene) and to their use in the treatment of dermatological disorders related to inflammation, in particular psoriasis. The present invention also relates to the use of said compounds or their mixture and the pharmaceutically acceptable addition salts thereof for the manufacture of a medicament for topical treatment of inflammation and inflammation-related skin disorders, particularly for topical treatment of psoriasis.

Also, the present invention relates to pharmaceutical formulations of 2,6-dimethoxynaphtalene or 2,7-dimethoxynaphtalene or their mixture (Endipalene) for use in the topical treatment of inflammation and inflammation-related skin disorders, particularly for topical treatment of psoriasis.

The object of this invention is to provide suitable formulations of 2,6-dimethoxynaphtalene or 2,7-dimethoxynaphtalene or their mixture (Endipalene) for the treatment of inflammation and inflammation-related skin disorders.

More particularly, the object of this invention relates to the use of said formulations for the manufacture of a medicament for the topical treatment of psoriasis.

With the term "formulations or pharmaceutical formulations" are intended formulations comprising 2,6-dimethoxynaphtalene or 2,7-dimethoxynaphtalene or their mixture (Endipalene) as active ingredient, in addition with other components and/or additives compatible with the pharmaceutical practice.

With the term "pharmaceutically acceptable acid addition salts" are intended those salts which from biological, manufacturing and formulation standpoint are compatible with the pharmaceutical practice.

According to this invention the formulations are suitable for topical delivery of active ingredients. The topical formulations suitable for the treatment of inflammation-related skin disorders are for example creams, lotions, mousses, sprays, emulsions, gels and ointments, which are manufactured according to methods commonly know in the art.

The formulations according to the present invention comprise respectively: from 0.01% to 0.5% of active ingredient, i.e. 2,6-dimethoxynaphtalene or 2,7-dimethoxynaphtalene or their mixture (Endipalene) (Formulation A) and from 5% to 10% of active ingredient, i.e. 2,6-dimethoxynaphtalene or 2,7-dimethoxynaphtalene or their mixture (Endipalene) (Formulation B).

In the treatment of psoriasis, medications with the least potential for adverse reactions are preferentially employed. If the treatment goal is not achieved, then therapies with greater potential toxicity may be used, but should be avoided as far as possible. In any case, as a first step, topical treatments, for example medicated ointments or creams, are applied to the skin.

The disadvantages of topical treatments are variably that they can often irritate normal skin, can be time consuming and awkward to apply, cannot be used for long periods, can stain clothing or have a strong odour. As a result, it is sometimes difficult for people to maintain the regular application of these medications. In addition, abrupt withdrawal of some topical agents, can cause an aggressive recurrence of the condition. This is known as a rebound of the condition.

Thus, also when an efficient topical treatment is used, a low concentration of the active ingredient should be employed, in order to avoid irritation of the skin. Furthermore, the treatment cannot be used for long periods, with the consequence that, as immediate side effect, the interruption of the topical treatment very often causes an increase and sometimes a complete resumption of the illness.

It is thus provided, according to the present invention, a formulation (Formulation B) comprising from 5% to 10% of active ingredient, i.e. 2,6-dimethoxynaphtalene or 2,7-dimethoxynaphtalene or their mixture (Endipalene).

This formulation has shown a very good and improved activity with respect to the formulation comprising Endipalene according to the prior art (1-2% of active ingredient), inducing a very fast and persistent reduction of the symptoms in patients affected by psoriasis. 20 Patients affected by psoriasis at a comparable level, were divided into two groups (a control group and a test group). Both groups were treated for 20 days, twice a day, with topical applications of respectively a formulation comprising 1-2% of Endipalene according to the prior art or with a formulation comprising 8% of Endipalene according to the present invention (Formulation B). The course of the psoriasis disease was observed in both groups and evaluated.

Test group shown an unexpected and significant reduction of the illness onset, evaluable in a greater extent if compared with the reduction observed for control group. Even though test group was treated with a formulation with a high concentration of Endipalene with respect to control group, the extent of the observed decrease of psoriasis symptoms can be evaluated as greater than the corresponding decrease that would be expected simply increasing the concentration of the active agent in the formulation. Thus, the surprisingly effect observed in test group, is not entirely due to the increase in the concentration of the active ingredient, but also to different and unexpected factors.

Table 1 shows the results obtained according to the above.

**TABLE 1**

| **DAYS OF TRATMENT** | **CONTROL GROUP** | **TEST GROUP** |
|---|---|---|
| **1** | n | n |
| **2** | n | p |
| **3** | p | p |
| **4** | p | m |
| **5** | p | m |
| **6** | m | s |
| **7** | m | s |
| **8** | m | c |
| **9** | m | c |
| **10** | m | c |

| | | |
|---|---|---|
| **n**-no reversion, **p**-low reversion, **m**-medium reversion, **s**-significant reversion, **c-**complete reversion. | | |

Always according to the present invention, it has been surprisingly found that a subsequent and extended treatment of patients (previously treated with Formulation B according to the invention) with a formulation (Formulation A) comprising from 0.01% to 0.5% of active ingredient, i.e. 2,6-dimethoxynaphtalene or 2,7-dimethoxynaphtalene or their mixture (Endipalene), resulted in an interesting maintaining of the reversion of the symptoms obtained with the main treatment.

This finding represents an interesting result, as the prolonged treatment of patients with formulation A according to the invention, allows to overcome the recurrence of the condition when the main treatment is interrupted. Thus it is possible to protract the pharmacological treatment for a long time, for example firstly applying the higher concentration formulation in order to obtain in a short time the best results, and then maintaining the obtained results applying the lower concentration formulation for a long time period, without any adverse side effect.

Always according to the present invention, the formulation comprising Endipalene in concentrations ranging from 0.01% to 0.5% of active ingredient (Formulation A), can be successfully used since the beginning as the sole pharmacological treatment of psoriasis. In fact, despite the quite low concentration of the active ingredient comprised in the formulation, a great positive effect has been observed when patients affected by psoriasis were treated for a medium - long period of time, for example 30-40 days.

In this case, 20 patients affected by psoriasis at a comparable level, were divided into two groups (a control group and a test group). Both groups were treated for 40 days, twice a day, with topical applications of respectively a formulation comprising 1-2% of Endipalene according to the prior art or a formulation comprising 0.3% of Endipalene according to the present invention (Formulation A). The course of the psoriasis disease was observed in both groups and evaluated.

Test group shown an unexpected and significant reduction of the illness onset, if compared with the reduction observed for control group. Even though test group was treated with a formulation with a low concentration of Endipalene with respect to control group, the extent of the observed decrease of psoriasis symptoms can be evaluated as very interesting, and also non corresponding to the decrease that would be expected simply lowering the concentration of the active agent in the formulation. Thus, there is a surprisingly effect observed in test group, certainly due to unexpected factors.

Table 2 shows the results obtained according to the above.

**TABLE 2**

| **DAYS OF TRATMENT** | **CONTROL GROUP** | **TEST GROUP** |
|---|---|---|
| **1** | n | n |
| **2** | n | n |
| **3** | p | n |
| **4** | p | p |
| **5** | p | p |
| **6** | m | p |
| **7** | m | p |
| **8** | m | m |
| **9** | m | m |
| **10** | m | m |

| | | |
|---|---|---|
| **n**-no reversion, **p**-low reversion, **m**-medium reversion, **s**-significant reversion, **c-**complete reversion. | | |

In the subsequent 30 days, both groups maintained a condition corresponding to a medium reversion of the symptoms (**m**).

According to the above, formulation A is effective in the treatment of psoriasis, is able to reduce symptoms, is suitable for long term treatments assuring a constant maintenance of the benefic effects and, at the same time, avoids side effects such as, for example, irritation of the skin.

All concentrations indicated in the present application are intended as weight percentages of the active ingredient on the total weight of the formulation/composition.

## Claims

1. Pharmaceutical formulation comprising from 0.01% to 0.5% by weight of 2,6-dimethoxynaphtalene or 2,7-dimethoxynaphtalene or their mixture as active ingredient on the total weight of the formulation, in addition with other components and/or additives compatible with the pharmaceutical practice.

2. Pharmaceutical formulation comprising from 5% to 10% by weight of 2,6-dimethoxynaphtalene or 2,7-dimethoxynaphtalene or their mixture as active ingredient on the total weight of the formulation, in addition with other components and/or additives compatible with the pharmaceutical practice.

3. Pharmaceutical formulation according to claim 1, **characterized in that** it comprises 0.3% by weight of 2,6-dimethoxynaphtalene or 2,7-dimethoxynaphtalene or their mixture as active ingredient on the total weight of the formulation.

4. Pharmaceutical formulation according to claim 2, **characterized in that** it comprises 8% by weight of 2,6-dimethoxynaphtalene or 2,7-dimethoxynaphtalene or their mixture as active ingredient on the total weight of the formulation.

5. Use of the formulation according to any of claims 1 to 4, for the manufacture of a medicament for topical treatment of inflammation and inflammation-related skin disorders.

6. Use of the formulation according to any of claims 1 to 4, for the manufacture of a medicament for topical treatment of psoriasis.

7. Pharmaceutical formulations according to any of claims 1 to 4, for use in the treatment of inflammation and inflammation-related skin disorders.

8. Pharmaceutical formulations according to any of claims 1 to 4, for use in the topical treatment of psoriasis.

9. Pharmaceutical formulations according to claims 1 to 4 **characterized in that** they are for topical treatment.

## Patentansprüche

1. Pharmazeutische Zubereitung, enthaltend 0,01 bis 0,5 Gew.-% 2,6-Dimethoxynaphtalen oder 2,7-Dimethoxynaphtalen oder Mischungen davon bezogen auf das Gesamtgewicht der Zubereitung als Wirkstoff, zusätzlich mit anderen Bestandteilen und/oder Zusätzen, die mit der pharmazeutischen Praxis vereinbar sind.

2. Pharmazeutische Zubereitung, enthaltend 5 bis 10 Gew.-% 2,6-Dimethoxynaphtalen oder 2,7-Dimethoxynaphtalen oder Mischungen davon bezogen auf das Gesamtgewicht der Zubereitung als Wirkstoff, zusätzlich mit anderen Bestandteilen und/oder Zusätzen, die mit der pharmazeutischen Praxis vereinbar sind.

3. Pharmazeutische Zubereitung gemäß Anspruch 1, **dadurch gekennzeichnet, dass** sie 0,3 Gew.-% 2,6-Dimethoxynaphtalen oder 2,7-Dimethoxynaphtalen oder Mischungen davon bezogen auf das Gesamtgewicht der Zubereitung als Wirkstoff enthält.

4. Pharmazeutische Zubereitung gemäß Anspruch 2, **dadurch gekennzeichnet, dass** sie 8 Gew.-% 2,6-Dimethoxynaphtalen oder 2,7-Dimethoxynaphtalen oder Mischungen davon bezogen auf das Gesamtgewicht der Zubereitung als Wirkstoff enthält.

5. Verwendung der Zubereitung gemäß irgendeinem der Ansprüche 1 bis 4 zur Herstellung eines Arzneimittels zur topischen Behandlung von entzündlichen und entzündungsähnlichen Hautkrankheiten.

6. Verwendung der Zubereitung gemäß irgendeinem der Ansprüche 1 bis 4 zur Herstellung eines Arzneimittels zur topischen Behandlung von Psoriasis.

7. Pharmazeutische Zubereitungen gemäß irgendeinem der Ansprüche 1 bis 4 zur Verwendung in der Behandlung von entzündlichen und entzündungsähnlichen Hautkrankheiten.

8. Pharmazeutische Zubereitungen gemäß irgendeinem der Ansprüche 1 bis 4 zur Verwendung in der Behandlung von Psoriasis.

9. Pharmazeutische Zubereitungen gemäß den Ansprüchen 1 bis 4, **dadurch gekennzeichnet, dass** sie für die topische Behandlung verwendet werden.

## Revendications

1. Formulation pharmaceutique comprenant 0,01 % à 0,5 % en poids de 2,6-diméthoxynaphtalène ou de 2,7-diméthoxynaphtalène ou leur mélange en tant qu'ingrédient actif du poids total de la formulation, en plus d'autres composants et/ou additifs compatibles avec la pratique pharmaceutique.

2. Formulation pharmaceutique comprenant 5 % à 10 % en poids de 2,6-diméthoxynaphtalène ou de 2,7-diméthoxynaphtalène ou leur mélange en tant qu'ingrédient actif du poids total de la formulation, en plus d'autres composants et/ou additifs compatibles avec la pratique pharmaceutique.

3. Formulation pharmaceutique selon la revendication 1, **caractérisée en ce qu'**elle comprend 0,3 % en poids de 2,6-diméthoxynaphtalène ou de 2,7-diméthoxynaphtalène ou leur mélange en tant qu'ingrédient actif du poids total de la formulation.

4. Formulation pharmaceutique selon la revendication 2, **caractérisée en ce qu'**elle comprend 8 % en poids de 2,6-diméthoxynaphtalène ou de 2,7-diméthoxynaphtalène ou leur mélange en tant qu'ingrédient actif du poids total de la formulation.

5. Utilisation de la formulation selon l'une quelconque des revendications 1 à 4, pour la production d'un médicament destiné au traitement topique d'une inflammation et de troubles cutanés associés à une inflammation.

6. Utilisation de la formulation selon l'une quelconque des revendications 1 à 4, pour la production d'un médicament destiné au traitement topique du psoriasis.

7. Formulations pharmaceutiques selon l'une quelconque des revendications 1 à 4, à utiliser dans le traitement d'une inflammation et de troubles cutanés associés à une inflammation.

8. Formulations pharmaceutiques selon l'une quelconque des revendications 1 à 4, à utiliser dans le traitement topique du psoriasis.

9. Formulations pharmaceutiques selon les revendications 1 à 4, **caractérisées en ce qu'**elles sont destinées au traitement topique.
